# Europäisches Patentamt

# European Patent Office

# Office européen des brevets

(19)

(11) Veröffentlichungsnummer: **0 206 142**
**B1**

(12) **EUROPÄISCHE PATENTSCHRIFT**

(45) Veröffentlichungstag der Patentschrift:
**31.08.88**

(51) Int. Cl.⁴: **C 07 C 69/533, C 07 C 67/333**

(21) Anmeldenummer: **86108022.4**

(22) Anmeldetag: **12.06.86**

(54) **Verfahren zur Herstellung von 4-Pentensäureestern.**

(30) Priorität: **14.06.85 DE 3521380**

(43) Veröffentlichungstag der Anmeldung:
**30.12.86 Patentblatt 86/52**

(45) Bekanntmachung des Hinweises auf die Patenterteilung:
**31.08.88 Patentblatt 88/35**

(84) Benannte Vertragsstaaten:
**BE CH DE FR GB IT LI NL**

(56) Entgegenhaltungen:
**EP-A-0 126 349**
**DE-A-3 040 432**

(73) Patentinhaber: **BASF Aktiengesellschaft, Carl-Bosch- Strasse 38, D-6700 Ludwigshafen (DE)**

(72) Erfinder: **Hoelderich, Wolfgang, Dr., Mannheimer Strasse 18 c, D-6710 Frankenthal (DE)**
Erfinder: **Fischer, Rolf, Dr., Bergstrasse 98, D-6900 Heidelberg (DE)**
Erfinder: **Maisch, Klaus- Dieter, Dr., Amselweg 14, D-6707 Schifferstadt (DE)**
Erfinder: **Lendle, Hubert, Dr., Ruedigerstrasse 41, D-6700 Ludwigshafen (DE)**

**Beschreibung**

Bei der Herstellung von Pentensäureestern durch Umsetzen von Butadien mit Kohlenmonoxid und Alkanolen in Gegenwart von Metallcarbonylkatalysatoren, wie es z. B. in der DE-A-3 040 432 beschrieben wird, erhält man ein Gemisch aus isomeren Pentensäureestern. Für weitere Umsetzungen z. B. zum Herstellen von δ-Formylvaleriansäureestern durch Hydroformylierung von Pentensäureester wird jedoch 4-Pentensäureester als Ausgangsverbindung bevorzugt. Es wurde deshalb schon versucht, 4-Pentensäureester durch Isomerisierung von isomeren Pentensäureester zu erhalten. Wie aus Bull. of the Chem. Soc. of Japan, Band 46, Seite 528 bekannt ist, erhält man durch Isomerisieren von 3-Pentensäuremethylester in Gegenwart von Cobaltcarbonylen jedoch vorwiegend 2-Pentensäuremethylester. Nach einer anderen in Tetrahedron Vol. 28, Seiten 5769-77 (1972) beschriebenen Arbeitsweise gelingt es zwar in Gegenwart eines Komplexes aus Rhodiumtriphenylphosphin und Zinnchlorid das Isomerengleichgewicht so zu verschieben, daß 4-Pentensäureester erhalten werden. Der dort verwendete Katalysator wird jedoch innerhalb weniger Stunden inaktiv.

In der EP-A-126 349 wird ein Verfahren zur Herstellung von 4-Pentensäureestern aus einem Pentensäureestergemisch durch Behandeln mit sauren Zeolithen, die einen Gehalt an Edelmetallen der achten Gruppe des Periodischen Systems haben, beschrieben.

Bei der Isomerisierung des Pentensäureesters liegen nach Erreichen des thermodynamischen Gleichgewichts fünf Isomere, nämlich 4-Pentensäureester, cis- und trans-3-Pentensäureester sowie cis- und trans-2-Pentensäureester vor, wobei das Gleichgewicht stark nach der Seite des trans-2-Pentensäureesters verschoben ist.

Es war deshalb die technische Aufgabe gestellt, ein Verfahren zur Herstellung von 4-Pentensäureestern aus isomeren Petensäureestern zur Verfügung zu stellen, bei dem die Katalysatoren über längere Zeit ihre Aktivität behalten und zudem die lineare Verschiebung der Doppelbindung zum 4-Pentensäureester bevorzugt verläuft und zudem möglichst wenig des schwer abtrennbaren cis-2-Pentensäureesters gebildet wird.

Diese technische Aufgabe wird gelöst in einem Verfahren zur Herstellung von 4-Pentensäureestern durch Isomerisierung, wobei man isomere Pentensäureester bei erhöhter Temperatur in Gegenwart von Zeolithen mit einem Gehalt an Metallen der achten Gruppe des Periodischen Systems behandelt und 4-Pentensäureester aus dem Reaktionsgemisch abdestilliert, dadurch gekennzeichnet, daß die Zeolithe einen Gehalt an Eisen, Cobalt und/oder Nickel haben.

Das neue Verfahren hat den Vorteil, daß die verwendeten Katalysatoren eine lange Lebensdauer haben und wohlfeiler sind. Das neue Verfahren hat ferner den Vorteil, daß bevorzugt eine lineare Verschiebung der Doppelbindung zu 4-Pentensäureester eintritt. Ein besonderer Vorteil des neuen Verfahrens ist es, daß die Bildung des schwer abtrennbaren cis-2-Pentensäureesters minimiert oder sogar völlig unterdrückt wird. Das anfallende Reaktionsgemisch ist leichter durch Destillation trennbar.

Vorteilhaft geht man von isomeren Pentensäureestern z. B. 2- oder 3-Pentensäureestern aus, die sich von Alkoholen mit bis zu 12 Kohlenstoffatomen herleiten. Besonders bevorzugt verwendet man isomere Pentensäurealkylester insbesondere von Alkoholen mit bis zu 4 Kohlenstoffatomen. Geeignete Pentensäureester sind beispielsweise 3-Pentensäuremethylester, 3-Pentensäureethylester 3-Pentensäurepropylester, 3-Pentensäurebutylester, 2-Pentensäuremethylester, 2-Pentensäureethylester sowie 2-Pentensäurepropylester. Geeignet sind auch Gemische aus isomeren Pentensäureestern, wie sie bei der Umsetzung von Butadien mit Kohlenmonoxid und Alkoholen in Gegenwart von Metallcarbonylen entsprechend DE-OS-3 040 432 erhalten werden. Besondere Bedeutung haben 3-Pentensäureester erlangt.

Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Silicium- und Aluminiumatome zu Sauerstoff beträgt 1 : 2. Die Elektrovalenz der Aluminium enthaltenen Tetraeder ist durch Einschluß von Kationen in den Kristall, z. B. durch Alkali- oder Wasserstoffionen ausgeglichen. Ein Kationenaustausch ist möglich. Die Räume zwischen den Tetraedern sind vor der Dehydration durch Trocknen bzw. Calcinieren von Wassermolekülen besetzt.

Entsprechend ihrer Struktur werden Zeolithe in verschiedene Gruppen unterteilt (siehe Ullmanns Encyclopädie der techn. Chemie, 4. Auflage, Band 24, S. 575 (1983)). So liegen bei der Mordenit-Gruppe Ketten oder bei der Chabasit-Gruppe Schichten aus Tetraedern, die Zeolith-Struktur haben, vor, während sich bei der Faujasit-Gruppe die Tetraeder zu Polyedern ordnen, z. B. in Form eines Kuboktaeders, der aus Vierringen bzw. Sechsringen aufgebaut ist. Bevorzugte Zeolithe sind A-, X- oder Y-Zeolithe, insbesondere Y-Zeolithe.

Die Zeolithe werden nach ihrer Isolierung in der Regel bei 100 bis 160°C, vorzugsweise 110°C, getrocknet und bei 450 bis 550°C, vorzugsweise 500°C, calciniert. Vorteilhaft werden sie mit einem Bindemittel im Gewichtsverhältnis 90 : 10 bis 40 : 60 zu Strängen oder Tabletten verformt. Als Bindemittel eignen sich z. B. Aluminiumoxide vorzugsweise Boehmit, hochdisperses Aluminiumoxid, amorphe Aluminiumsilikate mit einem Verhältnis von $SiO_2$ zu $Al_2O_3$ von 25 : 75 bis 90 : 5, insbesondere 75 : 25, Siliciumdioxid, insbesondere hochdisperses Siliciumdioxid, Gemische aus hochdispersem Siliciumdioxid und hochdispersem Aluminiumoxid, hochdisperses Titandioxid sowie Ton. Nach der Verformung werden die Extrudate oder Preßlinge bei einer Temperatur von 100 bis 160°C für einen Zeitraum von 2 bis 24 Stunden getrocknet und anschließend bei einer Temperatur von 450 bis 550°C für einen Zeitraum von 2 bis 24 Stunden calciniert.

Man erhält auch vorteilhafte Katalysatoren, wenn der isolierte Zeolith direkt nach der Trocknung verformt wird und erstmals nach der Verformung calciniert wird. Die hergestellten Zeolithe können aber auch in reiner

# 0 206 142

Form ohne Binder als Stränge oder Tabletten verwendet werden. Die Verformung erfolgt hierbei unter Zusatz von Verstrangungshilfs- bzw. Peptisierungsmitteln wie Hexamethylcellulose, Stearinsäure, Kartoffelstärke, Ameisensäure, Essigsäure, Oxalsäure, Salpetersäure, Ammoniak, Amine, Silicoester, Graphit oder deren Gemische.

Liegt der Zeolith aufgrund der Art seiner Herstellung nicht in der bevorzugten acide H-Form vor, sondern z. B. in der Natriumform, so kann diese vorteilhaft durch Ionenaustausch mit Ammoniumionen und anschließende Calcinierung oder durch Behandlung mit Säuren vollkommen oder partiell in die H-Form überführt werden.

Die verwendeten Zeolithe haben einen Gehalt an Eisen, Cobalt und/oder Nickel. Vorteilhaft beträgt der Gehalt an den vorgenannten Metallen 0,1 bis 7,0 Gew.-%, bezogen auf Zeolithe. Besonders bewährt hat sich ein Gehalt von Cobalt. Ferner hat es sich als vorteilhaft erwiesen, wenn die verwendeten Katalysatoren neben den vorgenannten Metallen der achten Gruppe zusätzlich ein Gehalt an Alkalimetallionen wie Natrium- oder Kaliumionen, insbesondere Natriumionen, oder Erdalkalimetallionen wie Calcium- oder Bariumionen, insbesondere Calciumionen, haben. Der Gehalt an Alkali- und/oder Erdalkalimetallionen beträgt vorteilhaft 0,05 bis 3,5 Gew.-%, bezogen auf Zeolith.

Zweckmäßig führt man die Dotierung z. B. so durch, daß man verformte Zeolithe in einem Steigrohr vorlegt und bei 20 bis 100°C eine wäßrige und/oder aminhaltige Lösung eines Halogenids oder Nitrats der vorbeschriebenen Metalle darüberleitet. Ein derartiger Ionentausch kann z. B. an der Wasserstoff-, Amonium- oder Alkaliform des Zeoliths vorgenommen werden. Eine weitere Möglichkeit der Metallaufbringung auf Zeolithe ist gegeben, indem man diese mit einem Halogenid, einem Nitrat oder einem Oxid der vorbeschriebenen Metalle in wäßriger und/oder alkoholischer und/oder aminhaltiger Lösung imprägniert. Sowohl an einem Ionenaustausch als auch an eine Imprägnierung schließt sich zumindest ein Trockenvorgang und wahlweise eine abermalige Calcinierung an.

Eine mögliche Ausführungsform besteht z. B. darin, daß man Co(NO$_3$)$_2$ · 6 H$_2$O in Wasser löst. Mit dieser Lösung wird der verformte oder unverformte Zeolith in der Regel 1 bis 30 Minuten getränkt. Überschüssiges Wasser wird abdestilliert und anschließend der getränkte Zeolith bei einer Temperatur von 100 bis 160°C getrocknet und bei 450 bis 550°C calciniert. Dieser Tränkvorgang kann mehrmals hintereinander vorgenommen werden bis der gewünschte Metallgehalt erreicht ist.

Es ist auch möglich, reinen pulverförmigen Zeolith in einer wäßrigen Lösung von Cobaltnitrat bei 40 bis 500°C unter Rühren über einen Zeitraum z. B. bis zu 24 Stunden aufzuschlämmen. Nach Abfiltrieren und Trocknen sowie Calcinieren bei den vorgenannten Temperaturen kann das so gewonnene zeolithische Gut mit oder ohne Bindemittel zu Strängen oder Pellets weiterverarbeitet werden.

Ein Ionentausch des in der H-Form oder Ammonium-Form oder Natrium-Form vorliegenden Zeolithen kann z. B. so vorgenommen werden, daß man den mit und ohne Bindemittel verformten Zeolith in Strängen oder Pellets in einer Kolonne vorlegt und darüber eine wäßrige Lösung von Cobaltnitrat bei einer Temperatur von 30 bis 80°C im Kreislauf 15 bis 20 Stunden leitet. Anschließend wird mit Wasser ausgewaschen und bei den vorgenannten Temperaturen getrocknet und calciniert.

Bei Zeolithen, die mit den vorgenannten Metallen der achten Gruppe des Periodischen Systems dotiert sind, hat sich eine Nachbehandlung mit Wasserstoff z. B. bei einer Temperatur von 200 bis 500°C als vorteilhaft erwiesen. Vorteilhaft wird vor der Behandlung mit Wasserstoff der Katalysator langsam in Gegenwart molekularen Sauerstoffs oder solchen enthaltenden Gasen auf eine Temperatur von bis zu 350°C erhitzt. Zudem ist es vorteilhaft, wenn der verwendete Wasserstoff ein Gehalt an Olefinen mit 2 bis 4 Kohlenstoffatomen hat.

Eine weitere Möglichkeit der Modifizierung besteht darin, daß man den Zeolith verformt oder unverformt mit Säuren, wie Salzsäure, Flußsäure, Phosphorsäure und/oder mit Wasserdampf behandelt. Ferner kann durch partielle Verkokung die Aktivität des Katalysators für ein Selektivitätsoptimum eingestellt werden.

Die hier beschriebenen Katalysatoren können wahlweise als 2 bis 4 mm Stränge oder als Tabletten mit einem Durchmesser von 3 bis 5 mm oder als Pulver mit einer Teilchengröße von 0,1 bis 0,5 mm angewandt werden.

Bei der Isomerisierung hält man im allgemeinen eine Temperatur von 50 bis 300°C ein. Im allgemeinen führt man die Isomerisierung unter Atmosphärendruck durch. Je nach Art des verwendeten Esters können jedoch auch verminderter Druck oder erhöhter Druck z. B. bis zu 50 bar angewandt werden.

Es ist möglich, die Isomerisierung diskontinuierlich durchzuführen, indem man z. B. in einem Rührbehälter pulverförmigen Katalysator in dem Isomerengemisch suspendiert und für einen Zeitraum von 2 bis 20 Stunden auf 50 bis 250°C, insbesondere auf 100 bis 200°C, erhitzt.

Bevorzugt wird die Isomerisierung kontinuierlich durchgeführt. Dabei wird ein Reaktionsrohr mit verformtem Katalysator gefüllt und das isomere Pentensäureestergemisch mit einer mittleren Verweilzeit von 5 bis 100 Minuten bei einer Temperatur von 50 bis 250°C, insbesondere von 100 bis 200°C, vorzugsweise 100 bis 120°C, flüssig durch das Rohr gepumpt. Aus dem so erhaltenen Gemisch wird 4-Pentensäureester durch Destillation abgetrennt und das verbleibende Gemisch wiederum zurückgeführt.

Besonders bevorzugt wird die Isomerisierung in der Gasphase durchgeführt. Hierbei wird das verdampfte isomere Pentensäureestergemisch gegebenenfalls unter Mitverwendung eines inerten Trägergases wie Stickstoff über den Katalysator, z. B. im Wirbelbett, geleitet. Vorteilhaft hält man eine Temperatur von 150 bis 300°C, insbesondere von 160 bis 250°C, ein. Es hat sich als vorteilhaft erwiesen, wenn man eine Belastung WHSV (g Einsatzgemisch je g Katalysator und Stunde) von 0,1 bis 20 h$^{-1}$ einhält. In der Regel wird das anfallende dampfförmige Gemisch kondensiert und anschließend 4-Pentensäureester durch Destillation abgetrennt. Die verbleibenden Isomeren werden vorteilhaft wieder der Isomerisierung zugeführt.

3

Falls bei der erfindungsgemäßen Verwendung der zeolithischen Katalysatoren durch Koksabscheidung eine Desaktivierung eintritt, empfiehlt es sich, die Zeolithe durch Abtrennen der Koksablagerung mit Luft oder mit einem Gemisch aus Luft und Stickstoff bei einer Temperatur von 400 bis 550°C, insbesondere 490 bis 540°C zu regenerieren. Die Zeolithe erhalten dadurch ihre Anfangsaktivität zurück.

4-Pentensäureester eignen sich zur Herstellung von δ-Formylvaleriansäureester, einem Vorprodukt für die Herstellung von ε-Caprolactam, Hexandiol oder Adipinsäure.

Das verfahren sei an folgenden Beispielen veranschaulicht:

**Herstellung der Katalysatoren**

**Katalysator A**

Natrium-Y-Zeolith wird mit Boehmit im Gewichtsverhältnis 60 : 40 zu 2 mm Strängen verformt, bei 110°C getrocknet und bei 500°C 16 Stunden calciniert. Diese Stränge werden in einer Kolonne mit einer 20 gew.-%-igen Ammoniumchloridlösung im Gewichtsverhältnis 1 : 15 bei 80°C behandelt. Dieser Ionenaustausch wird solange wiederholt, bis der Natriumgehalt < 0,15 Gew.-% beträgt. Anschließend wird bei 110°C getrocknet und dann bei 500°C 5 Stunden calciniert. Die so behandelten Stränge werden mit einer Cobaltnitratlösung imprägniert, abermals bei 110°C getrocknet und bei 540°C 2 Stunden calciniert. Der Cobaltgehalt beträgt 3,3 Gew.-%.

**Katalysator B**

Natrium-Y-Zeolith wird mit Boehmit im Gewichtsverhältnis 60 : 40 zu 2 mm Strängen verformt, bei 110°C getrocknet und bei 500°C 16 Stunden calciniert. Diese Stränge werden in einer Kolonne mit 20 gew.-%-iger Calciumnitratlösung im Gewichtsverhältnis 1 : 15 bei 80°C 2 Stunden behandelt. Anschließend wird bei 110°C getrocknet und bei 500°C 5 Stunden calciniert. Die so erhaltenen Stränge werden abermals in einer Kolonne mit einer Cobaltnitratlösung bei 80°C 2 Stunden behandelt, danach bei 130°C getrocknet und bei 540°C 2 Stunden calciniert. Der Katalysator B enthält 1,2 Gew.-% Cobalt, 0,5 Gew.-% Natrium und 0,1 Gew. -% Calcium.

**Katalysator C** (Vergleich)

Es wird als Katalysator ein Y-Zeolith, der mit 0,5 Gew.-% Palladium beladen ist, gemäß EP-A-126 349 verwendet.

**Beispiele 1 bis 3**

Ein Rohrreaktor von 0,6 cm Innendurchmesser und 90 cm Länge wird mit Katalysator beschickt und bei einer Temperatur von 180°C unter isothermen Bedingungen dampfförmiger 3-Pentensäuremethylester darüber geleitet. Das austretende Gasgemisch wird kondensiert und die Zusammensetzung gaschromatographisch bestimmt. Die näheren Bedingungen und Ergebnisse sind aus folgender Tabelle zu entnehmen.

**Tabelle**

|  | Beispiel | | Vergleichsbeispiel |
|---|---|---|---|
|  | 1 | 2 | 1 |
| Katalysator | A | B | C |
| Temperatur | 180°C | 180°C | 180°C |
| WHSV | 2 h⁻¹ | 2 h⁻¹ | 2 h⁻¹ |

4

**Produktzusammensetzung** Gew.-%

| 4-PSE | 4,4 | 10,4 | 10,7 |
|---|---|---|---|
| 3-PSE | 92,6 | 81,4 | 75,3 |
| trans | | | |
| 2-PSE | 3,0 | 8,2 | 13,1 |
| cis 2-PSE | 0 | 0 | 0,9 |

* PSE = Pentensäuremethylester

Andere Nebenprodukte waren unter den gewählten Reaktionsbedingungen nicht nachweisbar.

**Beispiel 3**

Man verfährt entsprechend Beispiel 2. Nach einer Reaktionsdauer von 6 Stunden fällt der Gehalt an 4-PSE auf 8,1 Gew.-%.

**Vergleichsbeispiel 2**

Man verfährt entsprechend Vergleichsbeispiel 1. Nach einer Reaktionsdauer von 6 Stunden fällt der Gehalt an 4-PSE auf 6,78 Gew.-%.

**Patentansprüche**

1. Verfahren zur Herstellung von 4-Pentensäureestern durch Isomerisierung, wobei man isomere Pentensäureester bei erhöhter Temperatur in Gegenwart von Zeolithen mit einem Gehalt an Metallen der achten Gruppe des Periodischen Systems behandelt und 4-Pentensäureester aus dem Reaktionsgemisch abdestilliert, dadurch gekennzeichnet, daß die Zeolithe einen Gehalt an Eisen, Cobalt und/oder Nickel haben.

2. Verfahren nach Anspruch 1, dadurch gekennzeichnet, daß die Zeolithe einen Gehalt an Eisen, Cobalt und/oder Nickel von 0,1 bis 7,0 Gew.-% haben.

3. Verfahren nach den Ansprüchen 1 und 2, dadurch gekennzeichnet, daß die Zeolithe einen Gehalt an Cobalt haben.

4. Verfahren nach den Ansprüchen 1 bis 3, dadurch gekennzeichnet, daß die Zeolithe zusätzlich einen Gehalt an Alkalimetall- und/oder Erdalkalimetallionen haben.

5. Verfahren nach den Ansprüchen 1 bis 4, dadurch gekennzeichnet, daß die Zeolithe einen Gehalt von 0,05 bis 3,5 Gew.-% an Alkalimetall- und/oder Erdalkalimetallionen haben.

6. Verfahren nach den Ansprüchen 1 bis 5, dadurch gekennzeichnet, daß man die Isomerisierung in der Gasphase bei einer Temperatur von 150 bis 300°C durchführt.

**Claims**

1. A process for the preparation of a 4-pentenoate by isomerization, in which isomeric pentenoates are treated at elevated temperatures in the presence of a zeolite which contains metals from group VIII of the periodic table and the 4-pentenoate is distilled off from the reaction mixture, wherein the zeolite contains iron, cobalt, and/or nickel.

2. A process as claimed in claim 1, wherein the zeolite contains from 0,1 to 7,0 % by weight of iron, cobalt and/or nickel.

3. A process as claimed in claims 1 and 2, wherein the zeolite contains cobalt.

4. A process as claimed in any of claims 1 to 3, wherein the zeolite additionally contains alkali metal and/or alkaline earth metal ions.

5. A process as claimed in any of claims 1 to 4, wherein the zeolite contains from 0,05 to 3,5 % by weight of alkali metal and/or alkaline earth metal ions.

6. A process as claimed in any of claims 1 to 5, wherein the isomerization is carried out in the gas phase at from 150 to 300°C.

**Revendications**

1. Procédé de préparation d'esters d'acide 4-penténique par isomérisation, en traitant des esters d'acide penténique isomères à température élevée en présence de zéolithes à teneur en métaux du huitième groupe du système périodique et en distillant du mélange de réaction l'ester d'acide 4-penténique, caractérisé par le fait que les zéolithes ont une teneur en fer, cobalt et/ou nickel.

2. Procédé selon la revendication 1, caractérisé par le fait que les zéolithes ont une teneur en fer, cobalt, et/ou nickel, de 0,1 à 7,0 % en poids.

3. Procédé selon les revendications 1 et 2 caractérisé par le fait que les zéolithes ont une teneur en cobalt.

4. Procédé selon les revendications 1 à 3 caractérisé par le fait que les zéolithes ont en outre une teneur en ions métal alcalin et/ou métal alcalino-terreux.

5. Procédé selon les revendications 1 à 4 caractérisé par le fait que les zéolithes ont une teneur de 0,05 à 3,5 % en poids d'ions métal alcalin et/ou métal alcalino-terreux.

6. Procédé selon les revendications 1 à 5 caractérisé par le fait qu'on effectue l'isomérisationen phase gazeuse à une température de 150 à 300°C.